(19) Europäisches Patentamt
European Patent Office
Office européen des brevets

(11) EP 4 324 818 A1

(12) EUROPEAN PATENT APPLICATION
published in accordance with Art. 153(4) EPC

(43) Date of publication:
**21.02.2024 Bulletin 2024/08**

(21) Application number: **22899143.6**

(22) Date of filing: **29.11.2022**

(51) International Patent Classification (IPC):
***C07C 59/01*** (2006.01) ***C12P 3/00*** (2006.01)
***C12P 7/52*** (2006.01) ***C01F 11/46*** (2006.01)

(52) Cooperative Patent Classification (CPC):
(C-Sets available)
**C07C 51/43; C01F 11/46; C07C 51/02; C07C 51/412; C12P 3/00; C12P 7/52;** Y02P 10/20

(Cont.)

(86) International application number:
**PCT/KR2022/019074**

(87) International publication number:
**WO 2023/096461 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2021 KR 20210167307**
**29.11.2021 KR 20210167409**
**28.11.2022 KR 20220161896**

(71) Applicant: **LG Chem, Ltd.**
**Seoul 07336 (KR)**

(72) Inventors:
- **HAN, Sang Won**
  **Daejeon 34122 (KR)**
- **AHN, Sangbum**
  **Daejeon 34122 (KR)**
- **JEONG, Yongbok**
  **Daejeon 34122 (KR)**
- **JUNG, Woochul**
  **Daejeon 34122 (KR)**
- **KIM, Si Min**
  **Daejeon 34122 (KR)**
- **KIM, Jimyeong**
  **Daejeon 34122 (KR)**
- **KIM, Taeho**
  **Daejeon 34122 (KR)**
- **KANG, Donggyun**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) PROCESS FOR RECOVERING ALKALI METAL SULFATE

(57) The present invention relates to a process of recovering an alkali metal sulfate, comprising: forming an organic acid fermentation liquid containing an alkali metal salt of organic acid; and adding sulfuric acid to the organic acid fermentation liquid to form and recover an alkali metal sulfate, wherein the alkali metal sulfate has a radioactivity concentration index of 1 or less, and to an alkali metal sulfate crystal comprising a predetermined peak in an X-ray diffraction spectrum (XRD) and having a radioactivity concentration index of 1 or less.

EP 4 324 818 A1

【FIG. 2】

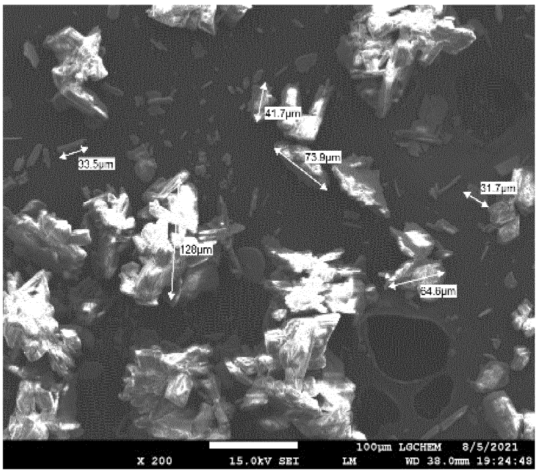


(52) Cooperative Patent Classification (CPC): (Cont.)

C-Sets
**C07C 51/02, C07C 59/01;**
**C07C 51/412, C07C 59/01;**
**C07C 51/43, C07C 59/01**

**Description**

## FIELD OF THE INVENTION

CROSS-REFERENCE TO RELATED APPLICATION(S)

This application claims the benefit of Korean Patent Application No. 10-2021-0167307, filed on November 29, 2021, Korean Patent Application No. 10-2021-0167409, filed November 29, 2021, and Korean Patent Application No. 10-2022-0161896, filed November 28, 2022, the disclosures of which are herein incorporated by reference in their entirety.
The present invention relates to a process of recovering an alkali metal sulfate and an alkali metal sulfate crystal.

## BACKGROUND OF THE INVENTION

Radon-222 is a natural radioactive substance that exists everywhere in living space, which is a colorless and tasteless gaseous substance that is produced from repeated radioactive decay of natural radionuclides (uranium-238, etc.) that naturally exist in soil and rocks, and is known as a human carcinogen that causes lung cancer.
However, natural radioactive substances contained in chemical gypsum, which is used as a raw material for cement and gypsum board, not only increase the radon concentration and increase the radiation exposure to workers, but also are used as construction materials, so that the occurrence of lung cancer due to exposure to radon has become a social problem.
In the past, a method of obtaining phosphate byproduct gypsum from rock phosphate, a method of obtaining byproduct gypsum generated in the titanium smelting process using ilmenite, or the like has been known. In the case of mineral-derived gypsum, however, it may contain a radioactive element, and the gypsum obtained by this method contains an excessive amount of various types of impurities, which requires an additional refining process.
Therefore, there is a need to develop a method for providing an environmentally friendly gypsum that does not substantially contain radioactive nuclides or heavy metals.

## SUMMARY OF THE INVENTION

## TECHNICAL PROBLEM

It is an object of the present invention to provide a process capable of recovering high-purity alkali metal sulfate or gypsum having a very low level of radioactivity concentration index.
It is another object of the present invention to provide an alkali metal sulfate crystal having a very low level of radioactivity concentration index, containing a very low content of impurities, and having a predetermined peak in an X-ray diffraction spectrum.

## TECHNICAL SOLUTION

Provided herein is a process of recovering an alkali metal sulfate, comprising:

forming an organic acid fermentation liquid containing an alkali metal salt of organic acid; and
adding sulfuric acid to the organic acid fermentation liquid to form and recover an alkali metal sulfate,
wherein the alkali metal sulfate has a radioactivity concentration index of 0.5 or less.

Also provided herein is an alkali metal sulfate crystal comprising 5 to 10 peaks at a diffraction angle ($2\theta \pm 0.2°$) of 12 to 32 in an X-ray diffraction spectrum (XRD), wherein the alkali metal sulfate has a radioactivity concentration index of 1 or less.
Now, a process of recovering an alkali metal sulfate and an alkali metal sulfate crystal according to embodiments of the present invention will be described in more detail.
It should be understood that, unless steps included in a preparation method described herein are specified as being sequential or consecutive or otherwise stated, one step and another step included in the preparation method should not be construed as being limited to an order described herein. Therefore, it should be understood that an order of steps included in a preparation method can be changed within the range of understanding of those skilled in the art, and that, in this case, the incidental changes obvious to those skilled in the art fall within the scope of the present invention.
In the present invention, the alkali metal includes both alkali metal and alkali earth metal.
As used herein, the term "substituted or unsubstituted" means being unsubstituted or substituted with one or more substituent groups selected from the group consisting of deuterium; a halogen group; a nitrile group; a nitro group;

a hydroxy group; a carboxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent group to which two or more substituent groups of the above-exemplified substituent groups are linked. For example, "a substituent group in which two or more substituents are linked" may be a biphenylyl group. Namely, a biphenylyl group may be an aryl group, or it may be interpreted as a substituent group in which two phenyl groups are linked.

According to one embodiment of the present invention, there can be provided a process of recovering an alkali metal sulfate, comprising:

forming an organic acid fermentation liquid containing an alkali metal salt of organic acid; and
adding sulfuric acid to the organic acid fermentation liquid to form and recover an alkali metal sulfate,
wherein the alkali metal sulfate has a radioactivity concentration index of 0.5 or less.

The present inventors have found through experiments that an alkali metal sulfate obtained by adding sulfuric acid to an organic acid fermentation liquid containing an alkali metal salt of organic acid has a very low level of a radioactive concentration index and contains a very low content of impurities, and that the alkali metal sulfate can be efficiently recovered in a state of high purity through a very simple process, and completed the invention.

The alkali metal sulfate may contain radioactive elements in an amount that does not substantially exist, or may be recovered in a state where radioactivity is not substantially emitted. More specifically, the radioactivity concentration index of the alkali metal sulfate may be 1 or less, or 0.5 or less, or 0.1 or less, or 0.05 or less, or 0.01 or less, 0.008 or less, 0.007 or less, or 0.007 or less.

That is, in the case of phosphate byproduct gypsum using gypsum derived from minerals, it is known that the radioactivity concentration index is about 2.03, whereas the alkali metal sulfate recovered in the above embodiment may have a very low level of radioactivity concentration index.

The radioactivity concentration index may be obtained from radioactivity concentrations which are measured with respect to potassium-40 for 10,000 seconds, measured with respect to radium-226 for 10,000 seconds, measured with respect to uranium-238 for 10,000 seconds, and measured with respect to thorium-232 for 10,000 seconds, using a high-purity germanium gamma-ray nuclide detector. More specifically, the radioactivity concentration index of the following general formula 1 can be obtained based on the measured radioactivity concentration.

[General Formula 1: Radioactivity Concentration Index]

◈ Radioactivity Concentration Index

$$I = \frac{C_{Ra226}}{300\ Bq/kg} + \frac{C_{Th232}}{200\ Bq/kg} + \frac{C_{K40}}{3000\ Bq/kg}$$

- $C_{Ra226}$, $C_{Th232}$, $C_{K40}$ are respectively the radioactivity concentration (Bq/kg) of solid radium-226($^{226}Ra$), thorium-232($^{232}Th$) and potassium-40($^{40}K$)

Further, when sulfuric acid is added to mineral-derived gypsum ($Ca(OH)_2$, $CaCO_3$, etc.) as shown in the following Reaction Scheme 2 to prepare an alkali metal sulfate, for example, calcium sulfate ($CaSO_4$), not only does it contain radioactive elements as described above, but the mineral-derived gypsum has low solubility in solvents such as water, and a concentration difference depending on the volume occurs, resulting in a low reaction rate, which causes the problem that the alkali metal sulfate contains a large amount of impurities.

[Reaction Scheme 2]

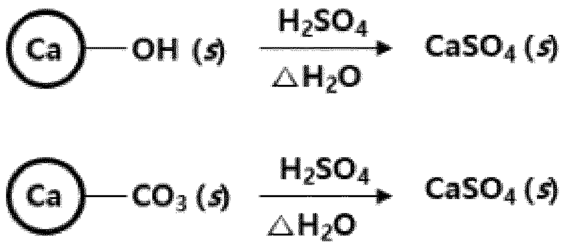


However, in the process of recovering the alkali metal sulfate according to one embodiment of the invention, sulfuric acid can be added to an alkali metal salt of organic acid to produce an alkali metal sulfate, for example, calcium sulfate ($CaSO_4$), as shown in the following Reaction Scheme 3. In addition, the alkali metal salt of organic acid has a high solubility in a solvent such as water, the concentration is uniformly dispersed within the reaction volume, and the reaction rate is uniformly high, so that the alkali metal sulfate may contain almost no impurities.

[Reaction Scheme 3]

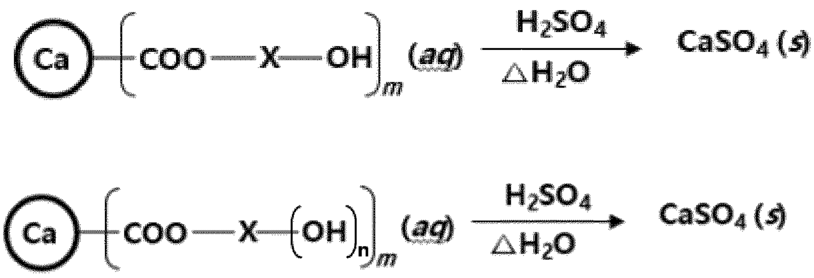


in Reaction Scheme 3,
the X is a carbonyl group (C=O); a substituted or unsubstituted $C_{1-10}$ alkylene; or a substituted or unsubstituted $C_{6-60}$ arylene,
the m is 1 or 2, and
the n is 0 or 1.

The organic acid means an organic compound having acidity, and for example, it may be an organic compound containing a carboxyl group, a sulfone group or the like. The organic acid is not limited thereto, but may be, for example, 3-hydroxypropionic acid, lactic acid, acetic acid, citric acid, salicylic acid, oxalic acid, tartaric acid, benzoic acid, and the like.

The alkali metal sulfate may be an alkali metal sulfate or an alkali metal sulfate hydrate. More specific examples of the alkali metal sulfate include calcium sulfate or calcium sulfate hydrate.

The alkali metal sulfate may contain 70 wt.% or more, 71 wt.% or more and 99 wt.% or less, 72 wt.% or more and 96 wt.% or less of gypsum dihydrate based on 100 wt.% of the alkali metal sulfate. When the alkali metal sulfate contains too little gypsum dihydrate, filterability may not be good.

Further, the alkali metal sulfate may contain 25 wt.% or less, 0.10 wt.% or more and 24 wt.% or less, 0.15 wt.% or more and 23 wt.% or less of gypsum hemihydrate based on 100% by weight of the alkali metal sulfate. When the alkali metal sulfate contains too much gypsum hemihydrate, lumps may form and agitation may be difficult.

Meanwhile, the step of adding sulfuric acid to the organic acid fermentation liquid to form and recover an alkali metal sulfate may comprise filtering the precipitate from the organic acid fermentation liquid to which sulfuric acid has been added.

When the alkali metal salt of the organic acid contained in the organic acid fermentation liquid reacts with sulfuric acid, a slurry composition may be produced. For example, when the organic acid alkali metal salt is a calcium salt of 3-hydroxypropionic acid ($Ca(3HP)_2$), the calcium salt of 3-hydroxypropionic acid ($Ca(3HP)_2$), which is an alkali metal salt of organic acid, and sulfuric acid can be reacted as in the following Reaction Scheme 1 to produce a slurry

composition containing a precipitate containing $CaSO_4 \cdot 2H_2O$ and 3-hydroxypropionic acid as an organic acid. Furthermore, the precipitate can be filtered from the slurry composition to recover the organic acid contained in the filtrate.

[Reaction Scheme 1] $Ca(3HP)_2 + H_2SO_4 + 2H_2O$ -> $CaSO_4 \cdot 2H_2O + 2(3HP)$

The alkali metal sulfate contained in the precipitate and recovered is in a highly pure state containing a very low content of impurities, and can also be recovered in a crystalline form having a predetermined peak in an X-ray diffraction spectrum (XRD).

The alkali metal sulfate can be recovered as an alkali metal sulfate crystal containing 5 to 10, 6 to 9, or 8 peaks at a diffraction angle ($2\theta \pm 0.2°$) of 12 to 32 in an X-ray diffraction spectrum (XRD).

Alternatively, the alkali metal sulfate can be recovered as an alkali metal sulfate crystal containing 3 to 5 or 4 peaks at a diffraction angle ($2\theta \pm 0.2°$) of 12 to 24 and containing 3 to 5 or 4 peaks at a diffraction angle ($2\theta \pm 0.2°$) of 26 to 32 in an X-ray diffraction spectrum (XRD).

Alternatively, the alkali metal sulfate can be recovered as an alkali metal sulfate crystal containing two peaks at a diffraction angle ($2\theta \pm 0.2°$) of 12 to 15, containing two peaks at a diffraction angle ($2\theta \pm 0.2°$) of 21 to 24, containing three peaks at a diffraction angle ($2\theta \pm 0.2°$) of 26 to 30, and containing a single peak at a diffraction angle ($2\theta \pm 0.2°$) of 32 to 33.

More specifically, the alkali metal sulfate can be recovered as an alkali metal sulfate crystal containing peaks at a diffraction angle ($2\theta \pm 0.2°$) of 12, 15, 21, 23.8, 26, 29.5, 30, and 32 in an X-ray diffraction spectrum (XRD).

In addition, the alkali metal sulfate crystal may further have peaks at a diffraction angle ($2\theta \pm 0.2°$) of 10.8, 31.5, 33.5, 34.5, 36.2, 36.8, 37.5, 41, 42.5, 43.5, 46, 48, 48.5, 49.5, 50.5, 51.5, 54.5, 55.2 and 57 in an X-ray diffraction spectrum (XRD), along with the peaks in the X-ray diffraction spectrum (XRD).

Meanwhile, at the time point when sulfuric acid is added to the organic acid fermentation liquid, the temperature of the organic acid fermentation liquid may be 4°C to 80°C, more specifically, 4°C or more, 10°C or more, 15°C or more, 30°C or more, 35°C or more, 40°C or more, 45°C or more, 50°C or more, or 55°C or more, and may be 90°C or less, 80°C or less, 75°C or less, 70°C or less, or 65°C or less.

The particle size and moisture content of the precipitate may be affected by the temperature conditions of the step of adding the acid. When an acid is added to the organic acid fermentation liquid under the above-mentioned temperature conditions, the precipitate may have large particle size and low moisture content.

As mentioned above, a slurry composition containing a precipitate and an organic acid may be produced through the reaction represented by Reaction Scheme 1, and in such a step, a precipitate containing an alkali metal salt represented by the following structural formula 3 may be formed.

[Structural Formula 3] Cation(Anion) • $pH_2O$

in the structural formula 3, the Cation is the cation of the alkali metal salt, the Anion is the anion of the acid, and the p is the number of water molecules in a hydrate, which is an integer of 1 or more. The cation may be $Ca^{2+}$, and the anion may be $SO_4^{2-}$.

Further, the particle size of the precipitate may be 0.5 $\mu$m or more, or 1 $\mu$m or more, and may be 100 $\mu$m or less, 40 $\mu$m or less, 20 $\mu$m or less, or 10 $\mu$m or less. The precipitate satisfies the above-mentioned particle size, and thus, the moisture content of the precipitate can be lowered, which can increase the fluidity of a slurry containing the precipitate and facilitate filtration of the precipitates from the slurry. The particle size of the precipitate can be measured by a scanning electron microscopy (SEM).

In addition, the moisture content of the precipitate may be 150% or less, 130% or less, 110% or less, 100% or less, 80% or less, or 70% or less, and may be 1% or more, 10% or more, or 30% or more. If the moisture content of the precipitate is too high, the fluidity of the slurry containing the precipitate is lowered, which makes it difficult to proceed with the process, and makes it difficult to separate the precipitate from the slurry, thus reducing the efficiency of the recovery process.

On the other hand, the organic acid fermentation liquid contains an alkali metal salt of organic acid or an organic acid in a predetermined amount or more, and thus can ensure the reaction efficiency due to the addition of sulfuric acid. In addition, since the relative content can be increased compared to other byproducts, the purity and efficiency can be further increased during the recovery of alkali metal salt sulfate.

For example, the organic acid fermentation liquid may contain an alkali metal salt or organic acid or an organic acid in an amount of 10 g/L or more, more specifically, 20 g/L or more, 30 g/L or more, 40 g/L or more, or 100 g/L or more, 150 g/L or more, 200 g/L or more, or 300 g/L or more, or 600 g/L or less, 500 g/L or less, 400 g/L or less, or 350 g/L or less.

When adding sulfuric acid to the organic acid fermentation liquid, the concentration and addition amount of the acid may be determined in consideration of the amount of an alkali metal salt of organic acid or an organic acid contained

in the organic acid fermentation liquid. For example, when adding sulfuric acid to the organic acid fermentation liquid, the equivalent of sulfuric acid added may be 0.5 to 2.0 times, or 0.8 to 1.5 times the total equivalent of the alkali metal salt of the organic acid or the organic acid contained in the organic acid fermentation liquid.

In addition, 0.1M to 10M sulfuric acid may be added in an amount of 10% or 500% of the volume of the organic acid fermentation liquid. Alternatively, when adding sulfuric acid to the organic acid fermentation liquid, the content of the sulfuric acid added may be 20 wt.% or more, 30 wt.% or more, and 80 wt.% or less, 70 wt.% or less, 60 wt.% or less, or 50 wt.% or less based on the total 100 wt.% of the alkali metal salt of the organic acid or the organic acid contained in the organic acid fermentation liquid.

When the content of the acid added is too small, the content of the alkali metal sulfate recovered is reduced, or the process efficiency is lowered. When the content of the acid added is too large, the concentration of unreacted cations and anions becomes too high, which may result in the generation of excessive byproducts and may adversely affect the progress of subsequent processes.

Meanwhile, the process of recovering an alkali metal sulfate according to one embodiment of the invention may comprise recovering an organic acid formed when adding sulfuric acid to an organic acid fermentation liquid containing an alkali metal salt of organic acid.

That is, according to the process of recovering an alkali metal sulfate of one embodiment of the invention, high-purity organic acids can be efficiently recovered together with alkali metal sulfates.

More specifically, the step of adding sulfuric acid to the organic acid fermentation liquid to form and recover an alkali metal sulfate may comprise filtering the precipitate from the organic acid fermentation liquid to which sulfuric acid has been added and recovering the organic acid from the filtered product.

The precipitate can be filtered from the slurry composition using a filtration flask, a vacuum pump, or the like, and an alkali metal sulfate and the like can be recovered from the precipitate, and the filtered precipitate can be washed to further recover the organic acid contained in the precipitate.

Therefore, the organic acid may be contained in the filtrate in which the precipitate has been filtered. Further, an organic acid may be contained in the washing solution used for washing the precipitate. However, since the washing liquid may contain other impurities in addition to the organic acid, the organic acid can be recovered from the filtrate in which the washing liquid have been filtered.

Components such as alkali metal sulfates contained in the precipitate can be confirmed through known methods such as X-ray fluorescence spectrometry (XRF), and the type and crystal form of the material can be confirmed using an X-ray diffraction analyzer (Bruker AXS D4-Endeavor XRD).

In one example, the recovery rate of organic acid in the step of recovering an organic acid provided by the present invention may be 40% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, but is not limited thereto. The recovery rate may be calculated based on the weight.

In the process of recovering an alkali metal sulfate according to one embodiment of the invention, the step of adding sulfuric acid to the organic acid fermentation liquid to form and recover an alkali metal sulfate may comprise adding sulfuric acid to a solution containing an alkali metal salt crystal of organic acid prepared from the organic acid fermentation liquid to from and recover the alkali metal sulfate.

For example, the step of adding sulfuric acid to the organic acid fermentation liquid to from and recover an alkali metal sulfate may further comprise forming and separating an alkali metal salt crystal of organic acid in the organic acid fermentation liquid in the presence of an alkali metal salt; preparing a solution containing the alkali metal salt crystal of organic acid; and adding sulfuric acid to the solution containing the alkali metal salt crystal of organic acid to form and recover the alkali metal sulfate.

Specifically, after the step of fermenting a bacterium strain having an organic acid-producing ability to form an organic acid fermentation liquid containing an alkali metal salt of organic acid, it is possible to form and separate the alkali metal salt crystal of organic acid in the organic acid fermentation liquid in the presence of an alkali metal salt.

The alkali metal salt crystal of organic acid may be formed by adding the alkali metal salt to the organic acid fermentation liquid, or the alkali metal salt may be formed in the presence of the alkali metal salt which is a neutralizing agent introduced in the neutral fermentation step, or may be formed by concentrating the organic acid fermentation liquid.

For example, when concentrating the organic acid fermentation liquid to form an alkali metal salt crystal of organic acid, the concentration may be carried out by evaporating a fermentation liquid (e.g., a liquid component of the fermentation liquid). The evaporation may be carried out by any means commonly available, and for example, it may be carried out by rotary evaporation concentration, evaporation concentration, vacuum concentration, concentration under reduced pressure, etc., but is not limited thereto.

The concentration of the alkali metal salt crystal of organic acid formed in the organic acid fermentation liquid or the concentrate of the fermentation liquid may be 20 g/L or more, 50 g/L or more, 100 g/L or more, 300 g/L or more,

350 g/L or more, 400 g/L or more, 450 g/L or more, or 500 g/L or more, and may be 900 g/L or less, 850 g/L or less, or 800 g/L or less.

Further, when the concentration of the alkali metal salt crystal of organic acid in the concentrate is higher than the water solubility of the alkali metal salt crystal of organic acid, the alkali metal salt crystal of organic acid can be more easily produced.

For example, when the alkali metal salt crystal of organic acid is $Ca(3HP)_2$, which is a crystal of the 3-hydroxypropionic acid, the water solubility of $Ca(3HP)_2$, is 450 g/L at room temperature, and thus, when the concentration of 3-hydroxypropionic acid in the concentrate exceeds 450 g/L, the formation of $Ca(3HP)_2$ crystal can be promoted. Further, the water solubility of $Mg(3HP)_2$, which is a crystal of 3-hydroxypropionic acid, is 250 g/L at room temperature, and thus, when the concentration of 3-hydroxypropionic acid in the concentrate exceeds 250 g/L, the formation of $Mg(3HP)_2$ crystal can be promoted.

The alkali metal salt crystal of organic acid can be formed from a concentrate satisfying the above-mentioned concentration even while containing the alkali metal salt. The alkali metal salt may include one or more cations selected from the group consisting of $Na^+$, $K^+$, $Mg^{2+}$ and $Ca^{2+}$, but when a cation of $Mg^{2+}$ or $Ca^{2+}$ or a salt thereof is used, 3-hydroxypropionic salt crystal can be formed more effectively. For example, the alkali metal salt may be one or more selected from the group consisting of NaOH, $NH_4OH$, KOH, $Ca(OH)_2$, CaO, $CaCO_3$ and $Mg(OH)_2$, and $Ca(OH)_2$, $Mg(OH)_2$ or a mixture thereof may be used to more effectively form the 3-hydroxypropionate crystal.

The alkali metal salt is added and remains in the process of producing the fermentation liquid, or it can be added in the process of forming the alkali metal salt crystal of organic acid in the concentrate containing 300 g/L or more of the organic acid. Further, the concentration of the alkali metal salt may be 10% to 100%, or 30% to 90% of the organic acid concentration, and for example, it can be present in the concentrate at a concentration of 10 to 900 g/L, 50 to 800 g/L, 100 to 700 g/L, or 200 to 600 g/L.

Further, the step of forming an alkali metal salt crystal of organic acid in the concentrate may further comprise contacting the concentrate with a nonsolvent. When contacting the concentrate with the nonsolvent, the alkali metal salt crystal of organic acid can be more easily formed. When the concentrate of the formed organic acid is brought into contact with a nonsolvent in the presence of the alkali metal salt, pure alkali metal salt crystal of organic acid is formed by controlling the crystal formation rate as compared to crystals formed at a high rate due to overconcentration, and then the crystal size can be increased. The alkali metal salt crystal of organic acid contains very low amounts of impurities inside the crystal, and have excellent filterability due to uniform crystal formation, so that not only it is easy to purify, but it is also excellent in shape stability and heating stability in the crystal state, thereby being able to efficiently mass-produce organic acid having high purity.

For example, when a nonsolvent is brought into contact with a concentrate of the formed organic acid in the presence of an alkali metal salt, an alkali metal salt of organic acid is produced, and as the concentration of the produced alkali metal salt of organic acid increases, fine crystals are formed, and solid-liquid phase separation occurs. After that, as crystallization proceeds, the alkali metal salt of organic acid grows into solid crystals (alkali metal salt crystal of organic acid), and the produced alkali metal salt crystal of organic acid contain impurities at a very low content while being separated from liquid impurities such as glycerol and 1,3-propanediol. At this time, the nonsolvent serves to promote crystallization of the alkali metal salt of organic acid, so that the solid-liquid separation ability is increased, and the alkali metal salt crystal of organic acid having higher purity can be produced.

The volume ratio between the concentrate and the nonsolvent may be determined by considering the concentration of organic acid or the volume of the concentrate and nonsolvent. For example, the concentrate and the nonsolvent may be used in a volume ratio of 1:0.5 to 1:20, or 1:0.5 to 1:10, or 1:0.8 to 1:8, or 1:1 to 1:5.

If the volume of the nonsolvent is too small compared to the concentrate, the concentration at which crystals are formed is high, and the rate of crystal formation is increased, so that pure crystal particles are not formed slowly and irregular crystal particles are rapidly formed. Further, the solid-liquid separation ability is low, which may make it difficult to separate liquid impurities from the alkali metal salt of organic acid to be purified. In addition, if the volume of the nonsolvent is too large compared to the concentrate, the formed crystals may be dissolved and dissolved in some cases due to the solubility of the nonsolvent, and the time in the crystal filtration process after crystal formation increases, and the amount of waste liquid increases, which may be uneconomical.

The nonsolvent may include an alcohol-based nonsolvent, a ketone-based nonsolvent, a nitrile-based nonsolvent, or a mixture of two or more thereof, and more specifically, at least one alcohol-based nonsolvent may be used.

The ketone-based nonsolvent may include one or more selected from the group consisting of acetone, methyl ethyl ketone, cyclohexanone, diethyl ketone, acetophenone, methyl isobutyl ketone, methylisoamylketone, isophorone and di-(isobutyl)ketone. The alcohol-based nonsolvent may include one or more selected from the group consisting of methanol, ethanol, allyl alcohol, 1-propanol, 2-propanol, 1-butanol, 2-butanol, isobutanol, benzyl alcohol, cyclohexanol, diacetone alcohol, ethylene glycol monoethyl ether, diethylene glycol monomethyl ether, diethylene glycol monoethyl ether, ethylene glycol monobutyl ether, diethylene glycol monobutyl ether, 2-methoxyethanol and 1-decanol. The nitrile-based nonsolvent may include one or more selected from the group consisting of acetonitrile, propionitrile, butyronitrile,

valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile.

The step of contacting the concentrate with a nonsolvent to form a 3-hydroxypropionate crystal may be carried out at a temperature of 0°C or more, 15°C or more, 30°C or more, 35°C or more, 40°C or more, 45°C or more, 50°C or more, or 55°C or more, and may be carried out at a temperature of 100°C or less, 90°C or less, 80°C or less, 75°C or less, 70°C or less, 65°C or less, or 0°C to 100°C.

At this time, the temperature may be adjusted to the above range by adding a non-solvent at the above-described temperature to the concentrate, or by heating or cooling in a state in which the concentrate and the nonsolvent are mixed.

The alkali metal salt crystal of organic acid may be a 3-hydroxypropionate crystal, and may be the form shown in the following structural formula 1 or structural formula 2. That is, the 3-hydroxypropionate crystal may include 3-hydroxypropionate in the form shown in the structural formula 1 or structural formula 2.

In the structural formula 1 and structural formula 2, Cation means a cation, 3HP means 3-hydroxypropionic acid that binds to the cation, n is the number of 3HP that binds to the cation and means an integer of 1 or more, and in the structural formula 2, m is the number of water molecules that binds to Cation(3HP)n in the hydrate, which is an integer of 1 or more. The cation may be, for example, $Na^+$, $Mg^{2+}$ or $Ca^{2+}$, but in the case of $Mg^{2+}$ or $Ca^{2+}$, 3-hydroxypropionate crystal may be formed more effectively.

[Structural Formula 1] $Cation(3HP)_n$

[Structural Formula 2] $Cation(3HP)_n \cdot mH_2O$

The particle size distribution $D_{50}$ of the 3-hydroxypropionate crystal may be 20 $\mu$m or more, 25 $\mu$m or more, 30 $\mu$m or more, 35 $\mu$m or more, and 300 $\mu$m or less, 250 $\mu$m or less, 200 $\mu$m or less, 150 $\mu$m, 90 $\mu$m or less, 85 $\mu$m or less, 80 $\mu$m or less, 75 $\mu$m or less.

In addition, the particle size distribution $D_{10}$ of the 3-hydroxypropionate crystal may be 5 $\mu$m or more and 40 $\mu$m or less, 8 $\mu$m or more and 35 $\mu$m or less, 10 $\mu$m or more and 30 $\mu$m or less, and the particle size distribution $D_{90}$ of the 3-hydroxypropionate crystal may be 50 $\mu$m or more and 200 $\mu$m or less, 60 $\mu$m or more and 190 $\mu$m or less, 65 $\mu$m or more and 180 $\mu$m or less, 70 $\mu$m or more and 175 $\mu$m or less.

The particle size distributions $D_{50}$, $D_{10}$ and $D_{90}$ mean particle diameters at which cumulative volumes of particles reach 50%, 10% and 90%, respectively, in the particle size distribution curve of the particles, wherein the $D_{50}$, $D_{10}$ and $D_{90}$ can be measured using, for example, a laser diffraction method. The laser diffraction method can generally measure particle sizes ranging from a submicron range to several millimeters, and can obtain results with high reproducibility and high resolvability.

If the particle size distributions $D_{50}$, $D_{10}$ and $D_{90}$ of the 3-hydroxypropionate crystal is too large, impurities that must be removed during crystallization may be contained in the crystals, which results in a reduction in purification efficiency. If the particle size distributions are too small, the liquid permeability during filtration of the crystals may be lowered.

Meanwhile, the $(D_{90}-D_{10})/D_{50}$ of the 3-hydroxypropionate crystal may be 1.00 or more and 3.00 or less, 1.20 or more and 2.80 or less, 1.40 or more and 2.60 or less, or 1.60 or more and 2.40 or less.

Further, the 3-hydroxypropionate crystal may have a volume average particle size of 30 $\mu$m or more and 100 $\mu$m or less, 35 $\mu$m or more and 95 $\mu$m or less, or 40 $\mu$m or more and 90 $\mu$m or less, a number average particle size of 1 $\mu$m or more and 30 $\mu$m or less, 3 $\mu$m or more and 25 $\mu$m or less, or 5 $\mu$m or more and 20 $\mu$m or less, and a volume average particle size of 10 $\mu$m or more and 70 $\mu$m or less, 15 $\mu$m or more and 60 $\mu$m or less, or 20 $\mu$m or more and 55 $\mu$m or less.

If the volume average particle size, number average particle size, and volume average particle size of the 3-hydroxypropionate crystal are too large, impurities that must be removed during crystallization may be contained in the crystals, which results in a reduction in purification efficiency. If they are too small, the liquid permeability during filtration of the crystals may be lowered.

Further, the LW ratio (length to width ratio) and average LW ratio in the particle size distribution ($D_{10}$, $D_{50}$, $D_{90}$) of the 3-hydroxypropionate crystal are 0.50 or more and 3.00 or less, 0.70 or more, 2.80 or less, and 1.00 or more and 2.50 or more. If the LW ratio of the 3-hydroxypropionate crystals is too large, problems of fluidity and clogging may occur during transfer of the crystals, and if the LW ratio is too small, the liquid permeability during filtration of the crystals may be lowered.

The 3-hydroxypropionate crystal can measure the moisture content contained in the crystals by the Karl Fischer method, and the moisture content contained in the 3-hydroxypropionate crystal may be 200 ppm or more and 5000 ppm or less, 250 ppm or more and 4800 ppm or less, 300 ppm or more and 4600 ppm or less, or 350 ppm or more and 4400 ppm or less.

At this time, the moisture contained in the 3-hydroxypropionate crystal means the attached moisture contained between the crystals, rather than the crystal moisture (for example, $Ca(3HP)_2 \cdot 2H_2O$). Further, if the moisture content contained in the 3-hydroxypropionate crystal is too high, it may be recovered in the form of a slurry rather than a crystalline solid, or impurities may be contained in the water, which may cause a problem of a decrease in purity improvement.

In the recovery process according to one embodiment of the invention, as 3-hydroxypropionic acid is prepared through a process such as fermenting a bacterium strain having 3-hydroxypropionic acid-producing ability as described below, the 3-hydroxypropionate crystal may contain a radioactive carbon isotope ($^{14}C$).

The radioactive carbon isotope ($^{14}C$) contains about 1 atom per $10^{12}$ carbon atoms in earth's atmosphere and has a half-life of about 5700 years, and carbon stocks can become abundant in the upper atmosphere due to nuclear reactions in which cosmic rays and normal nitrogen ($^{14}N$) participate. Meanwhile, in fossil fuels, isotopes have broken long ago, so that the $^{14}C$ ratio may be substantially zero. When bio-derived raw materials are used as the 3-hydroxypropionic acid raw material, or fossil fuels are used together with it, the content of radioactive carbon isotopes (pMC; percent modern carbon) and the content of bio-carbon contained in 3-hydroxypropionic acid can be measured according to the standard of ASTM D6866-21.

After carbon atoms contained in the compound to be measured is made into graphite form or carbon dioxide gas form, and can be measured, for example, by the mass spectrometer, or can be measured according to liquid scintillation analysis method. At this time, the two radioactive isotopes can be separated using an accelerator for separating $^{14}C$ ions from $^{12}C$ ions together with the mass spectrometer, and the content and the content ratio can be measured by a mass spectrometer.

The 3-hydroxypropionate crystal has a radiocarbon isotope content of 20 pMC (percent modern carbon) or more, 50 pMC or more, 90 pMC or more, 100 pMC or more, and a biocarbon content of 20 wt.% or more, 50 wt.% or more, 80 wt.%, 90 wt.%, or 95 wt.%, as measured according to the standard of ASTM D6866-21.

The radiocarbon isotope ratio (pMC) means the ratio of the radiocarbon isotope ($^{14}C$) contained in the 3-hydroxypropionic acid crystals to the radiocarbon isotope ($^{14}C$) of the current standard reference material, and it can be greater than 100% because the nuclear test program of the 1950s is still in effect and not extinguished.

Further, the content of biocarbon means the content of biocarbon relative to the total carbon content contained in the 3-hydroxypropionate crystal. As this value is larger, it may correspond to an environmentally friendly compound.

Meanwhile, if the radiocarbon isotope content (pMC) and biocarbon content of the 3-hydroxypropionate crystal are too low, the environmental friendliness is reduced, which may not be seen as a bio-derived material.

The crystalline state of the 3-hydroxypropionate crystal can be confirmed through peaks and the like in an X-ray diffraction (XRD) graph.

For example, the 3-hydroxypropionate crystal may exhibit peaks between crystal lattices in the $2\theta$ value range of 8 to 22° during X-ray diffraction (XRD) analysis.

For example, when the concentrate contains magnesium hydroxide ($Mg(OH)_2$), and the formed 3-hydroxypropionate crystal is $Mg(3HP)_2$, peaks between the crystal lattices due to the bond between 3-hydroxypropionic acid and magnesium may appear in the $2\theta$ value range of 8 to 15° during X-ray diffraction (XRD) analysis for $Mg(3HP)_2$. Such peaks show different results from the X-ray diffraction (XRD) analysis results for magnesium hydroxide ($Mg(OH)_2$) or magnesium sulfate ($Mg(SO_4)$). As a result of the X-ray diffraction (XRD) analysis, it can be confirmed that when a specific peak appears in the $2\theta$ value range of 8 to 15°, $Mg(3HP)_2$ crystal is formed.

Specifically, during X-ray diffraction (XRD) analysis for $Mg(3HP)_2$, 3 or more, 4 or more, or 5 or more peaks may appear in the $2\theta$ value range of 8 to 15°, and for example, peaks may appear in the $2\theta$ value range of 8.2 to 9.3°, 9.5 to 11.0°, 11.2 to 12.7°, 12.9 to 13.3°, and 13.5 to 14.8°, respectively.

Further, when calcium hydroxide ($Ca(OH)_2$) is contained in the concentrate and the 3-hydroxypropionate crystal formed therefrom are $Ca(3HP)_2$, peaks between the crystal lattices due to the bond between 3-hydroxypropionic acid and calcium may appear in the $2\theta$ value range of 10 to 22° during X-ray diffraction (XRD) analysis for $Ca(3HP)_2$. Such peaks show different results from the X-ray diffraction (XRD) analysis results for calcium hydroxide ($Ca(OH)_2$) or calcium sulfate ($Ca(SO_4)$). As a result of the X-ray diffraction (XRD) analysis, it can be confirmed that when a specific peak appears in the $2\theta$ value range of 10 to 22°, $Ca(3HP)_2$ crystal is formed.

Specifically, during X-ray diffraction (XRD) analysis for $Ca(3HP)_2$, 3 or more, 5 or more, 7 or more or 9 or more peaks may appear in the $2\theta$ value range of 10 to 22°, and for example, peaks may appear in the $2\theta$ value range of 10.0 to 11.0°, 11.1 to 11.6°, 11.6 to 12.5°, 12.7 to 13.6°, 13.8 to 16.0°, 17.0 to 18.0°, 19.0 to 19.8°, 20.2 to 21.2°, or 21.5 to 22.0°, respectively.

Meanwhile, the incident angle ($\theta$) means the point at which a first differential value (slope of the tangent line, dy/dx) is 0 on a graph where the horizontal axis (x-axis) in the x-y plane is a value of two times the incident angle ($2\theta$) of the incident X-ray, and the vertical axis (y-axis) in the x-y plane is a diffraction intensity, wherein as the value ($2\theta$) of two times the angle of incidence of the incident X-ray, which is the horizontal axis (x-axis), increases in the positive direction, the first differential value (slope of the tangent line, dy/dx) of two times ($2\theta$) the incident angle of X-rays, which is the horizontal axis (x-axis), with respect to the diffraction intensity, which is the vertical axis (y-axis), changes from a

positive value to a negative value.

Further, the 3-hydroxypropionate crystal may have a distance (d value) between atoms in the crystals derived from X-ray diffraction (XRD) analysis of 1.00 Å or more and 15.00 Å or less, 1.50 Å or more and 13.00 Å or less, 2.00 Å or more and 11.00 Å or less, 2.50 Å or more and 10.00 Å or less.

For example, when the 3-hydroxypropionate crystal is $Mg(3HP)_2$, a distance (d value) between atoms in the crystals of the peak appearing in the 2θ value range of 8 to 15° may be 1.00 Å or more and 15.00 Å or less, 2.00 Å or more and 13.00 Å or less, 4.00 Å or more and 11.00 Å or less, 5.50 Å or more and 10.00 Å or less.

Further, when the 3-hydroxypropionate crystal is $Ca(3HP)_2$, the distance (d value) between atoms in the crystals of the peak appearing in the 2θ value range of 10 to 22° may be 1.00 Å or more and 15.00 Å or less, 2.00 Å or more and 13.00 Å or less, 3.00 Å or more and 10.00 Å or less, 3.40 Å or more and 9.00 Å or less, or 4.00 Å or more and 8.50 Å or less.

Further, the 3-hydroxypropionate crystal may have a glass transition temperature of -55°C or more and -30°C or less, a melting point of 30°C or more and 170°C or less, and a crystallization temperature of 25°C or more and 170°C or less.

The glass transition temperature, melting point, and crystallization temperature may be measured by a differential scanning calorimetry (DSC) for the 3-hydroxypropionate crystal, wherein the heating rate during measurement may be 1 to 20°C/min. Further, the 3-hydroxypropionate crystal ate may have a glass transition temperature of -55°C or more and -30°C or less, -50°C or more and -35°C or less, or - 45°C or more and -40 °C or less. Further, the melting point of the 3-hydroxypropionate crystal may be 30°C or more and 170°C or less, 31°C or more and 160°C or less, 32°C or more and 150°C or less. Further, the crystallization temperature of the 3-hydroxypropionate crystal may be 25°C or more and 170 °C or less, 27°C or more and 160°C or less, or 30°C or more and 150°C or less. In addition, the crystallization stability section of the 3-hydroxypropionate crystal may be -40°C to 150°C.

The purity of 3-hydroxypropionate contained in the 3-hydroxypropionate crystal may be calculated as a percentage (%) of the mass of the compound having the structural formula 1 and/or structural formula 2 relative to the mass of the total crystal recovered. For example, the purity of 3-hydroxypropionate contained in the 3-hydroxypropionate crystal may be 70% or more, 80% or more, 90% or more, 70 to 99.9%, 80 to 99.9%, 90 to 99.9%, 70 to 99%, 80 to 99%, or 90 to 99%, but is not limited thereto.

After the step of forming and separating an alkali metal salt crystal of organic acid in the organic acid fermentation liquid in the presence of an alkali metal salt, a solution containing the alkali metal salt crystal of organic acid can be prepared.

As mentioned above, as the alkali metal salt crystal of organic acid is produced in the fermentation process, the concentrate in which the fermentation liquid has been concentrated may contain a large amount of impurities such as bacterium strains, carbon sources, and alkali metal salts in addition to the alkali metal salt crystal of organic acid. However, in the process of recovering 3-hydroxypropionic acid according to one embodiment of the invention, the impurities can be removed by solid-liquid separation and recovery of an alkali metal salt crystal of organic acid from the concentrate, and the impurities may not be contained even in a solution prepared by dissolving the separated alkali metal salt crystal of organic acid in a solvent such as distilled water.

Therefore, when sulfuric acid is added to the concentrate without separating the alkali metal salt crystal of organic acid from the concentrate, a large amount of impurities is contained in the concentrate, which may make it difficult to finally recover high-concentration and high-purity organic acid.

For example, the alkali metal salt crystal of organic acid can be separated from the concentrate using a filtration flask, a vacuum pump, or the like, and the separated alkali metal salt crystal of organic acid can be dissolved in a solvent such as distilled water to produce an aqueous solution of alkali metal salt crystal of organic acid.

The solution containing the alkali metal salt crystal of organic acid can ensure reaction efficiency due to the addition of the acid only when an alkali metal salt crystal of organic acid contains at a predetermined concentration or more. In addition, since the relative content can be increased compared to other byproducts, the purity and efficiency can be further increased during the recovery of organic acid. For example, the aqueous solution containing an alkali metal salt crystal of organic acid may contain an alkali metal salt crystal of organic acid or an organic acid in an amount of 10 g/L or more, and more specifically, 20 g/L or more, 30 g/L or more, 40 g/L or more, or 100 g/L or more, 150 g/L or more, 200 g/L or more, 300 g/L or more, or 600 g/L or less, 500 g/L or less, 400 g/L or less, or 350 g/L or less.

If the concentration of an alkali metal salt crystal of organic acid contained in the solution containing alkali metal salt crystal of organic acid is too low, the concentration of organic acid finally recovered may be lowered, and if the concentration of the alkali metal salt crystal of organic acid is too high, organic acid may be precipitated, or the solid phase concentration after precipitation may be too high, and thus, the recovery rate may be reduced due to a decrease in fluidity.

After the step of preparing a solution containing alkali metal salt crystal of organic acid, sulfuric acid can be added to the solution containing alkali metal salt crystal of organic acid to form and recover the alkali metal sulfate.

A cation exchange resin column can also be used in the step of recovering the organic acid.

Specifically, the process may comprise contacting the formed solution containing organic acid with a cation exchange resin column; and recovering organic acid from a solution contacted with the cation exchange resin column.

The cation exchange resin column serves to convert organic acid salt present in a solution containing organic acid to organic acid, whereby organic acid having higher purity can be separated and recovered with high efficiency.

Meanwhile, the cation exchange resin may be a polymer containing a functional group capable of ion exchange in its matrix, wherein the ion-exchangeable functional groups introduced into the matrix can be in the acid form capable of exchanging hydrogen cation ($H^+$), such as -COOH, -SOOH, and -POOH.

Further, polystyrene, acrylic polymer or the like can be used as the matrix of the cation exchange resin, and various matrixes such as homopolymers, co-polymers, block polymers and random copolymers can be used.

The particles of the cation exchange resin may have a spherical or irregular shape, and the particle size distribution range of the cation exchange resin may be 0.3 mm to 1.2 mm.

The exchange efficiency of the cation exchange resin may be 1.2 eq to 2.0 eq, or 1.6 eq to 1.9 eq, taking into account the stability of the cation exchange resin and the degree of ion desorption within the cation exchange resin.

In addition, the cation exchange resin may be used alone or in combination of two or more types, and the exchange efficiency varies depending on the type of the resin used, and thus, the resin can be selected in consideration of the particle size, shape, exchanger, and the like.

Meanwhile, the resin volume of the cation exchange resin column may be 2.5 BV or more, 10 BV or more, or 12 BV or more. The resin volume (BED VOLUME; BV) means the volume occupied by the filler in the column. If the resin volume content of the cation exchange resin column is too low, exchange efficiency decreases during ion exchange, and if the resin volume content is too high, the ion exchange time increases, which may be uneconomical.

Meanwhile, in the process of recovering the alkali metal sulfate, the step of forming an organic acid fermentation liquid containing an alkali metal salt of organic acid comprises adding an alkali metal or an alkali metal salt while fermenting a bacterium strain having an organic acid-producing ability in the presence of a carbon source.

For example, when the organic acid is 3-hydroxypropionic acid, the bacterium strain having 3-hydroxypropionic acid-producing ability may include, but is limited to, genes encoding at least one or two proteins selected from the group consisting of glycerol dehydratase and aldehyde dehydrogenase.

In one example, the 3-hydroxypropionic acid-producing strain may further include a gene (gdrAB) encoding glycerol dehydratase reactivator (GdrAB). In one example, the 3-hydroxypropionic acid-producing strain may be a bacterium strain that is further capable of biosynthesizing vitamin B12.

The glycerol dehydratase may be encoded by dhaB (GenBank accession no. U30903.1) gene, but is not limited thereto. The dhaB gene may be an enzyme derived from *Klebsiella pneumonia,* but is not limited thereto. The gene encoding the glycerol dehydratase may include a gene encoding dhaB1, dhaB2 and/or dhaB3. The glycerol dehydratase protein and the gene encoding it may include mutations in the gene and/or amino acid sequence within the range of maintaining an enzyme activity that decomposes glycerol to 3-hydroxypropanal (3-HPA) and water ($H_2O$).

The gene (aldH) encoding the aldehyde dehydrogenase (ALDH) may be, for example, aldH (GenBank Accession no. U00096.3; EaldH) gene derived from *Escherichia coli* or E. *coli* K12 MG1655 cell line, puuC gene derived from *Klebsiella pneumoniae,* and/or KGSADH gene from *Azospirillum brasilense,* but is not limited thereto. The aldehyde dehydrogenase protein and the gene encoding it may include mutations in the gene and/or amino acid sequence within the range of maintaining the activity for producing 3-hydroxypropionic acid from 3-hydroxypropanal.

The medium for producing the fermentation liquid can be selected without limitation within the range for the purposes for producing 3-hydroxypropionic acid. In one example, the medium may contain glycerol as a carbon source. In another example, the medium may be crude glycerol and/or pretreated crude glycerol, but is not limited thereto. In one example, the production medium may further include vitamin B12.

Further, the fermentation may be a weakly acidic fermentation or a neutral fermentation, for example, it may be maintained in the pH range of 3 to 8, 3.5 to 8, 4.5 to 7.5, or 6.5 to 7.5 during fermentation, but is not limited thereto. The pH range can be appropriately adjusted as needed.

When the fermentation is a neutral fermentation, the alkali metal salt may be added for the fermentation. The alkali metal salt may include $Mg^{2+}$, $Ca^{2+}$ or a mixture thereof. Further, the alkali metal salt may be $Ca(OH)_2$or $Mg(OH)_2$ , but is not limited thereto.

The recovery process according to one embodiment of the invention may further include removing (separating) cells from the fermentation liquid; purifying and/or decolorizing the fermentation liquid and/or the fermentation liquid from which cells has been removed; and/or filtering the fermentation liquid and/or the fermentation liquid from which cells have been removed, after the step of producing the 3-hydroxypropionic acid fermentation liquid.

Removal (separation) of the cells may be carried out by selecting methods known in the art without limitation within the range of the purpose of cell (strain) removal. In one example, the separation of the cells may be carried out by centrifugation.

The step of purifying and/or decolorizing the fermentation liquid and/or the fermentation liquid from which cells have been removed can be carried out by selecting methods known in the art without limitation within the range of the

purpose of purification of the fermentation liquid. For example, the step can be carried out by mixing activated carbon with the fermentation liquid and then removing the activated carbon, but is not limited thereto.

The step of filtering the fermentation liquid and/or the fermentation liquid from which cells have been removed can be carried out by selecting methods known in the art without limitation within the range of the purposes of removal of solid impurities, removal of proteins and/or materials with hydrophobic functional groups, and/or decoloration. For example, the step can be carried out by filter filtration and/or activated carbon filtration methods, but is not limited thereto.

In the recovery process according to one embodiment of the invention, as organic acid is prepared through a process such as fermenting a bacterium strain having organic acid-producing ability, the alkali metal salt of organic acid may contain a radioactive carbon isotope ($^{14}C$).

The radioactive carbon isotope ($^{14}C$) contains about 1 atom per $10^{12}$ carbon atoms in earth's atmosphere and has a half-life of about 5700 years, and carbon stocks can become abundant in the upper atmosphere due to nuclear reactions in which cosmic rays and normal nitrogen ($^{14}N$) participate. Meanwhile, in fossil fuels, radioactive carbon isotopes have broken long ago, so that the $^{14}C$ ratio may be substantially zero. When bio-derived raw materials are used as the organic acid raw material, or fossil fuels are used together, the content of radioactive carbon isotopes (pMC; percent modern carbon) and the content of bio-carbon contained in an organic acid or an alkali metal salt of organic acid can be measured according to the standard of ASTM D6866-21.

After carbon atoms contained in the compound to be measured is made into graphite form or carbon dioxide gas form, the content can be measured, for example, by a mass spectrometer, or can be measured according to a liquid scintillation analysis method. At this time, the two isotopes can be separated using an accelerator for separating $^{14}C$ ions from $^{12}C$ ions together with the mass spectrometer, and the content and the content ratio can be measured by a mass spectrometer.

The organic acid or the alkali metal salt of organic acid may have a radiocarbon isotope content of 20 pMC (percent modern carbon) or more, 50 pMC or more, 90 pMC or more, or 100 pMC or more, and a biocarbon content of 20 wt.% or more, 50 wt.% or more, 80 wt.%, 90 wt.%, or 95 wt.%, as measured according to the standard of ASTM D6866-21.

The radiocarbon isotope ratio (pMC) means the ratio between the radiocarbon isotope ($^{14}C$) contained in the organic acid or the alkali metal salt of organic acid to the radiocarbon isotope ($^{14}C$) of the current standard reference material, and it can be greater than 100% because the nuclear test program of the 1950s is still in effect and not extinguished.

Further, the content of biocarbon means the content of biocarbon relative to the total carbon content contained in the organic acid or the alkali metal salt of organic acid. As this value is larger, it may correspond to an environmentally friendly compound.

Meanwhile, if the radiocarbon isotope content (pMC) and biocarbon content of the organic acid or the alkali metal salt of organic acid are too low, the environmental friendliness is reduced, which may not be seen as a bio-derived material.

Meanwhile, according to another embodiment of the invention, there can be provided an alkali metal sulfate crystal comprising 5 to 10 peaks at a diffraction angle ($2\theta\pm0.2°$) of 12 to 32 in an X-ray diffraction spectrum (XRD), wherein the alkali metal sulfate has a radioactivity concentration index of 0.5 or less.

The alkali metal sulfate crystal provided has a very low level of radioactivity concentration index and contains a very low content of impurities, and thus can be efficiently recovered in a state of high purity even through a very simple process.

An alkali metal sulfate crystal having a crystal characteristic defined as a peak at a diffraction angle ($2\theta\pm0.2°$) in the X-ray diffraction spectrum (XRD) is not only easy to purify, but also it is also easy to stabilize in the crystalline state and have excellent heating stability, as compared to amorphous materials, etc., whereby the alkali metal sulfate crystal may enable mass production of products having high purity.

The alkali metal sulfate crystal may include 5 to 10, 6 to 9, or 8 peaks at a diffraction angle ($2\theta\pm0.2°$) of 12 to 32 in an X-ray diffraction spectrum (XRD).

Alternatively, the alkali metal sulfate crystal may include 3 to 5 or 4 peaks at a diffraction angle ($2\theta\pm0.2°$) of 12 to 24 in an X-ray diffraction spectrum (XRD), and include 3 to 5, or 4 peaks at a diffraction angle ($2\theta\pm0.2°$) of 26 to 32.

Alternatively, the alkali metal sulfate crystal may include 2 peaks at a diffraction angle ($2\theta\pm0.2°$) of 12 to 15, include 2 peaks at a diffraction angle ($2\theta\pm0.2°$) of 21 to 24, include 3 peaks at a diffraction angle ($2\theta\pm0.2°$) of 26 to 30, and include 1 peak at a diffraction angle ($2\theta\pm0.2°$) of 32 to 33.

More specifically, the alkali metal sulfate crystal may include peaks at a diffraction angle ($2\theta\pm0.2°$) of 12, 15, 21, 23.8, 26, 29.5, 30 and 32 in an X-ray diffraction spectrum (XRD).

Further, the alkali metal sulfate crystal may have peaks at a diffraction angle ($2\theta\pm0.2°$) of 10.8, 31.5, 33.5, 34.5, 36.2, 36.8, 37.5, 41, 42.5, 43.5, 46, 48, 48.5, 49.5, 50.5, 51.5, 54.5, 55.2 and 57 in an X-ray diffraction spectrum (XRD), together with the peak in an X-ray diffraction spectrum (XRD).

Meanwhile, the incident angle ($\theta$) means the point at which a first differential value (slope of the tangent line,

dy/dx) is 0 on a graph where the horizontal axis (x-axis) in the x-y plane is a value of two times the incident angle (2θ) of the incident X-ray, and the vertical axis (y-axis) in the x-y plane is a diffraction intensity, wherein as the value (2θ) of two times the angle of incidence of the incident X-ray, which is the horizontal axis (x-axis), increases in the positive direction, the first differential value (slope of the tangent line, dy/dx) of two times (2θ) the incident angle of X-rays, which is the horizontal axis (x-axis), with respect to the diffraction intensity, which is the vertical axis (y-axis), changes from a positive value to a negative value.

The alkali metal sulfate crystal may contain radioactive elements in an amount that does not substantially exist, or may be recovered in a state in which radioactivity is not substantially emitted. More specifically, the radioactivity concentration index of the alkali metal sulfate crystal may be 1 or less, or 0.5 or less, or 0.1 or less, or 0.05 or less, or 0.01 or less, 0.008 or less, 0.007 or less, or 0.007 or less.

That is, in the case of gypsum derived from minerals, it is known that the radioactivity concentration index is about 2.03, whereas the alkali metal sulfate crystal recovered in the above embodiment may have a very low level of radioactivity concentration index.

The radioactivity concentration index may be obtained from radioactivity concentrations which are measured with respect to potassium-40 for 10,000 seconds, measured with respect to radium-226 for 10,000 seconds, measured with respect to uranium-238 for 10,000 seconds, and measured with respect to thorium-232 for 10,000 seconds, using a high-purity germanium gamma-ray nuclide detector. More specifically, the radioactivity concentration index of the following general formula 1 can be obtained based on the measured radioactivity concentration.

The alkali metal sulfate crystals may be alkali metal sulfate crystals or alkali metal sulfate hydrate crystals. More specific examples of the alkali metal sulfate crystals include calcium sulfate crystals or calcium sulfate hydrate crystals.

The alkali metal sulfate crystals may contain gypsum dihydrate in an amount of 70 wt.% or more, 71 wt.% or more and 99 wt.% or less, and 72 wt.% or more and 96 wt.% or less based on 100 wt.% of the alkali metal sulfate crystals. In addition, the alkali metal sulfate crystals may contain gypsum hemihydrate in an amount of 25 wt.% or less, 0.10 wt.% or more and 24 wt.% or less, 0.15 wt.% or more and 23 wt.% or less based on 100 wt.% of the alkali metal sulfate crystals.

Meanwhile, the alkali metal sulfate crystal may have a particle size of 0.5 $\mu$m to 100 $\mu$m, may have a particle size of 0.5 $\mu$m or more, 2.0 $\mu$m or more, or 3.0 $\mu$m or more, and may have a particle size of 100 $\mu$m or less, 50 $\mu$m or less, or 20 $\mu$m or less. The particle size of the alkali metal salt crystal may be defined as the particle size range that 90% or more, or 99% or more of the total alkali metal salt crystal have.

Further, the alkali metal sulfate crystal may have a particle size distribution $D_{50}$ of 0.5 $\mu$m to 50 $\mu$m, or 10 $\mu$m to 40 $\mu$m.

The particle size distributions $D_{50}$, $D_{10}$ and $D_{90}$ mean particle sizes at which cumulative volumes of particles reach 50%, 10% and 90%, respectively, in the particle size distribution curve of the particles, wherein the $D_{50}$, $D_{10}$ and $D_{90}$ can be measured using, for example, a laser diffraction method. The laser diffraction method can generally measure particle sizes ranging from a submicron range to several millimeters, and can obtain results with high reproducibility and high resolvability.

If the particle size distributions $D_{50}$, $D_{10}$ and $D_{90}$ of the 3-hydroxypropionate crystal is too large, impurities that must be removed during crystallization may be contained in the crystals, which results in a reduction in purification efficiency. If the particle size distributions are too small, the liquid permeability during filtration of the crystals may be lowered.

## ADVANTAGEOUS EFFECTS

According to the present invention, there can be provided a process capable of recovering high-purity alkali metal sulfate or gypsum having a very low level of radioactivity concentration index, and an alkali metal sulfate crystal having a very low level of radioactivity concentration index, containing a very low content of impurities, and having a predetermined peak in an X-ray diffraction spectrum peak.

The alkali metal sulfate crystal or gypsum or alkali metal sulfate crystal provided above contain substantially no radionuclides or heavy metals, so that not only they are harmless to the human body, and are easy to purify, but also they are excellent in shape stability and heating stability, thereby being able to mass-produce a product having high purity.

## BRIEF DESCRIPTION OF THE DRAWINGS

FIG. 1 shows the results of X-ray diffraction analysis of alkali metal sulfate crystals recovered in Example 1.
FIG. 2 shows a scanning electron microscope (SEM) cross-sectional photograph of alkali metal sulfate crystals

recovered in Example 1.

## DETAILED DESCRIPTION OF THE EMBODIMENTS

Hereinafter, the present invention will be described in more detail with reference to examples. However, the following examples are for illustrative purposes only, and are not intended to limit the scope of the present invention.

### Preparation Example 1: Preparation of bacterium strain for producing 3-hydroxypropionic acid

Recombinant vectors into which genes encoding glycerol dehydratase and aldehyde dehydrogenase, which are known to produce 3-hydroxypropionic acid (3HP) using glycerol as a substrate, were introduced were manufactured. The prepared recombinant vector was introduced into *E.coli* W3110 strain to prepare a 3-hydroxypropionic acid-producing strain.

More specifically, a BtuR gene encoding adenosyltransferase was cloned into plasmid pCDF containing a gene (dhaB) encoding glycerol dehydratase, a gene (aldH) encoding aldehyde dehydrogenase and a gene (gdrAB) encoding glycerol dehydratase reactivase. The resulting pCDF_J23101_dhaB_gdrAB_J23100_aldH_btuR vector was introduced into strain W3110 (KCCM 40219) by an electroporation method using an electroporation device (Bio-Rad, Gene Pulser Xcell) to prepare 3-hydroxypropionic acid-producing strain. The process of preparing the 3-hydroxypropionic acid-producing strain of Preparation Example 1 and the vectors, primers, and enzymes used were carried out with reference to Example 1 of Korean Unexamined Patent Publication No. 10-2020-0051375, which is incorporated herein by reference.

### Preparation Example 2: Preparation of $Ca(3HP)_2$ fermentation liquid

The 3-hydroxypropionic acid-producing strain prepared in Preparation Example 1 was fermented and cultured at 35°C in a 5L fermenter using unpurified glycerol as a carbon source to produce 3-hydroxypropionic acid. In order to prevent the lowering of pH due to the production of 3-hydroxypropionic acid, calcium hydroxide ($Ca(OH)_2$), which is an alkali metal salt, was added thereto to maintain the pH to be neutral during the fermentation.

After fermentation culture, cells were removed by centrifugation (4000 rpm, 10 minutes, 4°C), and primary fermentation liquid purification (primary purification) was performed using activated carbon. Specifically, activated carbon was added to the fermentation liquid from which bacterial cells were removed by centrifugation, the mixture was well mixed, and then centrifuged again to separate the activated carbon. Then, the fermentation liquid from which the activated carbon was separated was filtered with a vacuum pump through a 0.7 um filter paper to purify the 3-hydroxypropionic acid fermentation liquid. The concentration of 3-hydroxypropionic acid in the fermentation liquid after completion of the primary purification was a level of 75 g/L.

### Preparation Example 3: Preparation of $Ca(3HP)_2$ crystal

The 3-hydroxypropionic acid-producing strain prepared in Preparation Example 1 was fermented and cultured at 35°C in a 5L fermenter using unpurified glycerol as a carbon source to produce 3-hydroxypropionic acid. In order to prevent the lowering of pH due to the production of 3-hydroxypropionic acid, calcium hydroxide ($Ca(OH)_2$), which is an alkali metal salt, was added thereto to maintain the pH to be neutral during the fermentation.

After fermentation culture, cells were removed by centrifugation (4000 rpm, 10 minutes, 4°C), and primary fermentation liquid purification (primary purification) was performed using activated carbon. Specifically, activated carbon was added to the fermentation liquid from which bacterial cells were removed by centrifugation, the mixture was well mixed, and then centrifuged again to separate the activated carbon. Then, the fermentation liquid from which the activated carbon was separated was filtered with a vacuum pump through a 0.7 um filter paper to purify the 3-hydroxypropionic acid fermentation liquid.

The concentration of 3-hydroxypropionic acid in the fermentation liquid after completion of the primary purification was a level of 50 to 100 g/L, and the fermentation liquid was concentrated to a concentration of 600 g/L using a rotary evaporator (50°C, 50 mbar) to prepare a concentrate, and ethanol was added in an amount of two times the volume of the concentrate, and stirred (300 rpm) at room temperature to produce $Ca(3HP)_2$ crystals. At this time, the concentration of the alkali metal salt in the concentrate was 493.3 g/L (based on $Ca(OH)_2$). The resulting crystals were washed three times with ethanol (EtOH) and dried in an oven at 50°C to finally recover the crystals.

### Example 1

3.5 L of the fermentation liquid obtained in Preparation Example 2 was prepared (contains about 75 g/L of 3-hydroxypropionic acid, and about 91 g/L of 3-hydroxypropionic acid calcium salt), and heated up to a temperature of

60°C. Then, 294 mL of 5M sulfuric acid solution was added to the heated fermentation liquid at a uniform rate for 5 minutes, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. The $CaSO_4$ precipitate was dried in an oven at a temperature of 40°C for 20 hours. Finally, the dried $CaSO_4$ precipitate was obtained, and a filtrate containing 3-hydroxypropionic acid was obtained.

**Example 2**

0.5 L of the fermentation liquid obtained in Preparation Example 2 was prepared (containing about 79 g/L of 3-hydroxypropionic acid, and about 96.8 g/L of 3-hydroxypropionic acid calcium salt), and heated up to a temperature of 60°C. Then, 46 mL of 5M sulfuric acid solution was added to the fermentation liquid at a uniform rate for 5 minutes, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. The $CaSO_4$ precipitate was dried in an oven at a temperature of 40°C for 20 hours. Finally, the dried $CaSO_4$ precipitate was obtained, and a filtrate containing 3-hydroxypropionic acid was obtained.

**Example 3**

An aqueous solution containing 600 g/L of $Ca(3HP)_2$ crystal recovered in Preparation Example 3 was prepared, and stirred at a temperature of 25°C and 350 rpm for 10 minutes. 37 ml of 5M sulfuric acid solution was added to 77 ml of the aqueous solution at a uniform rate for 5 minutes, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. Then, a filtrate (A) before washing was obtained in a filtering flask, and the filtered $CaSO_4$ precipitate was washed with 30 ml of distilled water, and then filtered to obtain a filtrate (B) after washing. Then, the $CaSO_4$ precipitate was dried in an oven at a temperature of 60°C for 20 hours to finally obtain the dried $CaSO_4$ precipitate. In addition, filtrates (A) and (B) containing 3-hydroxypropionic acid were obtained.

**Example 4**

1.0 L of the fermentation liquid obtained in Preparation Example 2 was prepared (containing about 84 g/L of 3-hydroxypropionic acid, and about 103 g/L of 3-hydroxypropionic acid calcium salt), and heated up to a temperature of 60°C. Then, 185 mL of 5M sulfuric acid solution was added to the fermentation liquid at a uniform rate for 5 minutes, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. The $CaSO_4$ precipitate was dried in an oven at a temperature of 40°C for 20 hours. Finally, the dried $CaSO_4$ precipitate was obtained, and a filtrate containing 3-hydroxypropionic acid was obtained.

**Example 5**

1.0 L of the fermentation liquid obtained in Preparation Example 2 was prepared (containing about 122 g/L of 3-hydroxypropionic acid, and about 149.5 g/L of 3-hydroxypropionic acid calcium salt), and heated up to a temperature of 60°C. Then, 500 mL of 5M sulfuric acid solution was added to the fermentation liquid at a uniform rate for 5 minutes, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. The $CaSO_4$ precipitate was dried in an oven at a temperature of 40°C for 20 hours. Finally, the dried $CaSO_4$ precipitate was obtained, and a filtrate containing 3-hydroxypropionic acid was obtained.

**<Test Example>**

**1. Analysis of precipitate components**

The precipitate containing $CaSO_4$ was dried, and specific component analysis of the finally obtained product was performed using an X-ray fluorescence spectrometer (XRF).

Specifically, primary element chemical analysis was performed on a ThermoARL Advant'X Sequential XRF with Uniquant standardless software and loss on ignition (LOI) normalization (moisture content included in the normalization), the loss on ignition (LOI) value was measured in an electric furnace maintained at 975°C, and the crystal moisture was determined by the LOI difference in a drying oven at 250°C.

[Table 1]

| Component (wt.%) | SiO2 | $Al_2O_3$ | $Fe_2O_3$ | CaO | MgO | $SO_3$ | LOI | Crystal moisture |
|---|---|---|---|---|---|---|---|---|
| Example 1 | - | 0.51 | 0.35 | 33.93 | - | 51.38 | 12.93 | 12.4 |
| Example 3 | 0.53 | 0.68 | 0.42 | 31.19 | - | 52.83 | 13.44 | 12.38 |

Then, the type and crystal form of the material were measured for the finally obtained product using an X-ray diffraction analyzer (Bruker AXS D4-Endeavor XRD). Specifically, measurement was performed under the following conditions; the applied voltage was 40 kV, the applied current was 40 mA, Cu-Kα radiation (wavelength λ = 1.54184 Å) was irradiated, the range of 2theta measured was 10° to 90°, and it was scanned at intervals of 0.05°.

[Table 2]

| wt. % | Gypsums | | | | Minerals | | | | | | |
|---|---|---|---|---|---|---|---|---|---|---|---|
| | Gypsum dihydrate | Gypsum hemihydrate | Gypsum anhydrite | Subtotal | Quartz | dolomite | Magnesite | illite | chlorite | microcline | Pyrite |
| Example 1 | 48.29 | 46.50 | 2.48 | 97.24 | 0.29 | 0.39 | 0.21 | 1.26 | 0.13 | 0.44 | - |
| Example 3 | 1.56 | 42.57 | 51.69 | 95.82 | 0.16 | 0.78 | 0.41 | 0.27 | 2.56 | 4.18 | - |
| Example 4 | 95.77 | 0.19 | 0 | 95.96 | - | - | - | 2.07 | - | - | - |
| Example 5 | 72.04 | 23.65 | 2.12 | 97.81 | 0.07 | 0.22 | - | 1.04 | - | 0.83 | 0.03 |

On the other hand, FIG. 1 shows the results of X-ray diffraction analysis of alkali metal sulfate crystals recovered in Example 1, and the main peak values and intensities at the diffraction angle ($2\theta \pm 0.2°$) shown in the XRD analysis results are shown in Table 3 below.

[Table 3]

| Diffraction angle ($2\theta \pm 0.2°$) | 12 | 15 | 21 | 23.8 | 26 | 29.5 | 30 | 32 |
|---|---|---|---|---|---|---|---|---|
| Intensity (a.u.) | 82,000 | 5,000 | 25,000 | 11,000 | 3,500 | 23,000 | 5,000 | 4,000 |

Referring to Tables 1 to 3, it was confirmed that the precipitates obtained in Examples 1 and 3 contained 95.82% or more of gypsum dihydrate, gypsum hemihydrate and gypsum anhydrite based on the total content, contained almost no minerals such as calcite and dolomite, and also contained almost no impurities such as $SiO_2$.

**3. Measurement of radioactivity concentration**

The precipitate containing the $CaSO_4$ was dried, and 1 kg of the finally obtained product was filled in a Marinelli beaker. Using a high-purity germanium gamma ray nuclide detector (HPGe Gamma Spectroscopy, produced by Kenbera, USA, relative efficiency: 30%, applicable energy band: 1,460 KeV), the radioactivity concentration was measured with respect to potassium-40 for 10,000 seconds, measured with respect to radium-226 for 10,000 seconds, measured with respect to uranium-238 for 10,000 seconds, and measured with respect to thorium-232 for 10,000 seconds. Then, based on the measurement result, the radioactivity concentration index of the following general formula 1 was obtained.

On the other hand, in Table 4 of the academic paper, Econ, Environ, Geol., 44(1), 37-48, 2011, the radioactivity concentration of Phosphogypsum 1 sample was shown. In addition, such Phosphogypsum was obtained after producing phosphate fertilizer from phosphate stone, and the radioactive concentration index of the following general formula 1 was also obtained for 1 kg of Phosphogypsum (Comparative Example).

[General Formula 1: Radioactivity Concentration Index]

◈ Radioactivity Concentration Index

$$I = \frac{C_{Ra226}}{300\ Bq/kg} + \frac{C_{Th232}}{200\ Bq/kg} + \frac{C_{K40}}{3000\ Bq/kg}$$

- $C_{Ra226}$, $C_{Th232}$, $C_{K40}$ are respectively the radioactivity concentration (Bq/kg) of solid radium-226($^{226}Ra$), thorium-232($^{232}Th$) and potassium-40($^{40}K$)

[Table 4]

| | Radionuclide | | | Radioactivity concentration index |
|---|---|---|---|---|
| | potassium-40 (Bq/Kg) | radium-226 (Bq/Kg) | thorium-232 (Bq/Kg) | |
| Example 1 | 18.55 ± 0.85 | below minimum detectable concentration | below minimum detectable concentration | 0.0062 |
| Comparative Example | 8.6 ± 6 | 591.9 ± 13 | 11.3 ± 1 | 2.03 |

Referring to Table 4, it was confirmed that Example can efficiently provide high-purity gypsum that contained radioactive elements in an amount that did not substantially exist, or that did not substantially emit radioactivity. On the other hand, in the case of the Phosphogypsum board using a stone furnace derived from the mineral of Comparative Example, the radioactive concentration was high.

**4. Measurement of recovery rate of 3-hydroxypropionic acid**

The recovery rate of 3-hydroxypropionic acid obtained in Example was measured and calculated using a high-performance liquid chromatography (HPLC).

Specifically, the absolute amount(X) of 3-hydroxypropionic acid in the fermentation liquid obtained in Preparation Example 2 and the absolute amount(Y) of 3-hydroxypropionic acid in the filtrate were measured, and the recovery rate of 3-hydroxypropionic acid was calculated according to the following general formula 2.

[General Formula 2]

$$\text{Recovery rate of 3-hydroxypropionic acid (\%)} = (Y / X) * 100$$

[Table 5]

| | Recovery rate of 3-hydroxypropionic acid |
|---|---|
| Example 1 | 87 |

Referring to Table 5, it was confirmed that in Example, high-purity 3-hydroxypropionic acid can be efficiently produced while separating the alkali metal sulfate.

## Claims

**1.** A process of recovering an alkali metal sulfate, comprising:

forming an organic acid fermentation liquid containing an alkali metal salt of an organic acid; and
adding sulfuric acid to the organic acid fermentation liquid to form and recover an alkali metal sulfate,
wherein the alkali metal sulfate has a radioactivity concentration index of 0.5 or less.

**2.** The process of recovering an alkali metal sulfate according to claim 1, wherein:
the radioactivity concentration index is obtained from radioactivity concentrations which are measured with respect to potassium-40 for 10,000 seconds, measured with respect to radium-226 for 10,000 seconds, measured with respect to uranium-238 for 10,000 seconds, and measured with respect to thorium-232 for 10,000 seconds, using a high-purity germanium gamma-ray nuclide detector.

**3.** The process of recovering an alkali metal sulfate according to claim 1, wherein:
the alkali metal sulfate includes calcium sulfate or calcium sulfate hydrate.

**4.** The process of recovering an alkali metal sulfate according to claim 1, wherein:

the alkali metal sulfate contains 70 wt.% or more of gypsum dihydrate based on 100 wt.% of the alkali metal sulfate, and
the alkali metal sulfate contains 25 wt.% or less of gypsum hemihydrate based on 100 wt.% of the alkali metal sulfate.

**5.** The process of recovering an alkali metal sulfate according to claim 1, wherein:
the alkali metal sulfate is recovered as an alkali metal sulfate crystal having an X-ray diffraction spectrum (XRD) having 5 to 10 peaks at a diffraction angle ($2\theta \pm 0.2°$) of 12 to 32.

**6.** The process of recovering an alkali metal sulfate according to claim 1, wherein:
the alkali metal sulfate is recovered as a crystal of the alkali metal sulfate having a particle size of 0.5 $\mu$m to 100 $\mu$m.

**7.** The process of recovering an alkali metal sulfate according to claim 1, wherein:

the adding sulfuric acid to the organic acid fermentation liquid to form and recover an alkali metal sulfate further

comprises,
forming and separating an alkali metal salt crystal of the organic acid in the organic acid fermentation liquid in the presence of the alkali metal salt;
preparing a solution containing the alkali metal salt crystal of the organic acid; and
adding the sulfuric acid to the solution containing the alkali metal salt crystal of the organic acid to form and recover the alkali metal sulfate.

**8.** The process of recovering an alkali metal sulfate according to claim 7, wherein:
the organic acid fermentation liquid or the solution containing the alkali metal salt crystal of the organic acid contains 10 g/L to 600 g/L of the alkali metal salt of the organic acid or the organic acid.

**9.** The process of recovering an alkali metal sulfate according to claim 1, wherein: in the adding of sulfuric acid to the organic acid fermentation liquid, the equivalent of the sulfuric acid added is 0.5 to 2.0 times the total equivalent of the alkali metal salt of the organic acid or the organic acid contained in the organic acid fermentation liquid.

**10.** The process of recovering an alkali metal sulfate according to claim 1, further comprising:
recovering an organic acid formed when adding the sulfuric acid to the organic acid fermentation liquid containing the alkali metal salt of organic acid.

**11.** The process of recovering an alkali metal sulfate according to claim 1, wherein:

the forming of an organic acid fermentation liquid containing an alkali metal salt of an organic acid comprises, adding an alkali metal or the alkali metal salt while fermenting a bacterium strain having an organic acid-producing ability in the presence of a carbon source.

**12.** An alkali metal sulfate crystal having an X-ray diffraction spectrum (XRD) having 5 to 10 peaks at a diffraction angle ($2\theta \pm 0.2°$) of 12 to 32,
wherein the alkali metal sulfate has a radioactivity concentration index of 0.5 or less.

**13.** The alkali metal sulfate crystal according to claim 12, wherein:
the alkali metal sulfate includes calcium sulfate or calcium sulfate hydrate.

**14.** The alkali metal sulfate crystal according to claim 12, wherein:

the alkali metal sulfate contains 70 wt.% or more of gypsum dihydrate based on 100 wt.% of the alkali metal sulfate crystal, and
the alkali metal sulfate contains 25 wt.% or less of gypsum hemihydrate based on 100 wt.% of the alkali metal sulfate crystal.

**15.** The alkali metal sulfate crystal according to claim 12, wherein:
the alkali metal sulfate crystal has a particle size of 0.5 $\mu$m to 100 $\mu$m.

**16.** The alkali metal sulfate crystal according to claim 12, wherein:
the alkali metal sulfate crystal has a particle size distribution $D_{50}$ of 0.5 $\mu$m to 50 $\mu$m.

【FIG. 1】

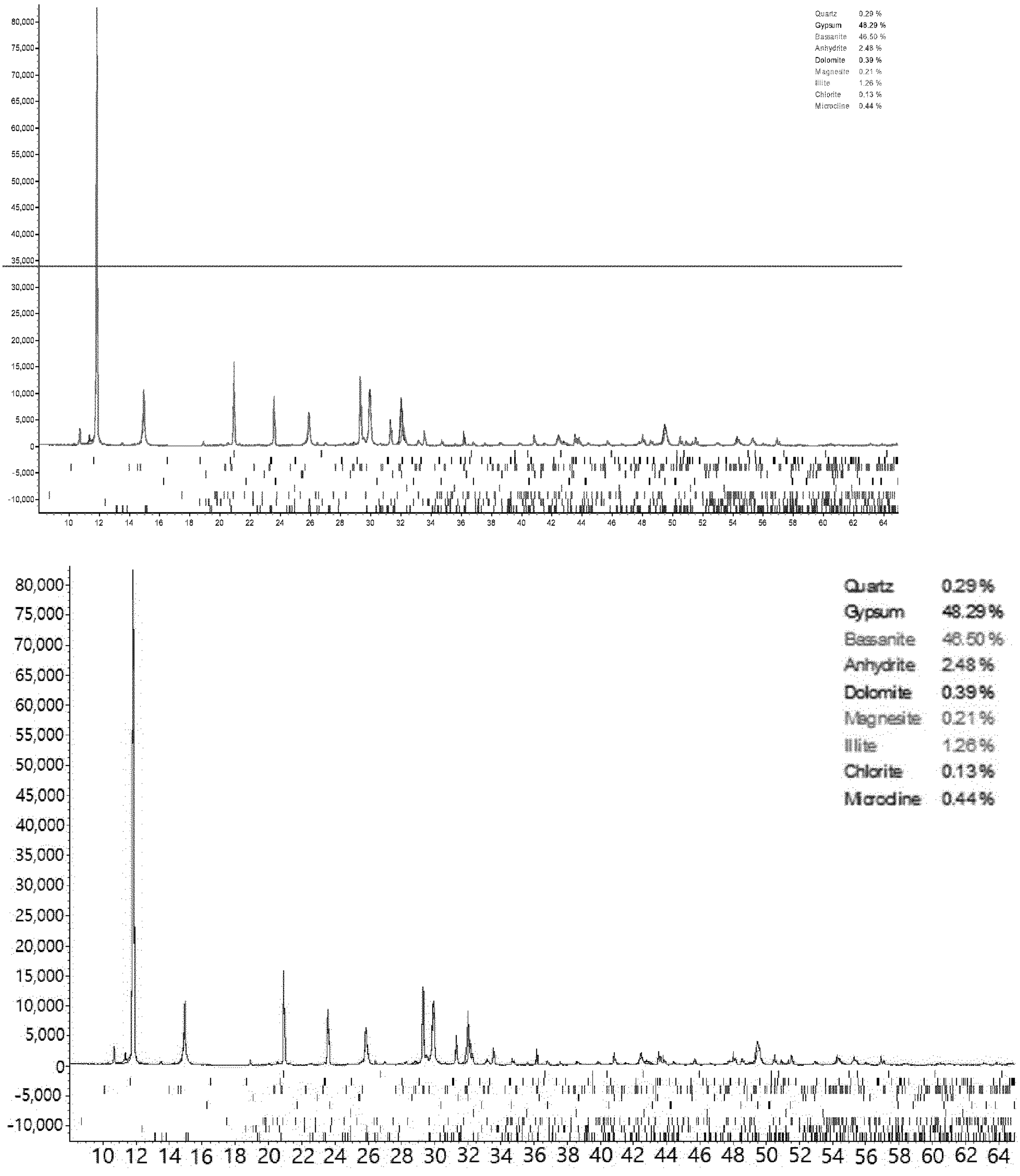
Quartz 0.29 %
Gypsum 48.29 %
Bassanite 46.50 %
Anhydrite 2.48 %
Dolomite 0.39 %
Magnesite 0.21 %
Illite 1.26 %
Chlorite 0.13 %
Microcline 0.44 %

【FIG. 2】

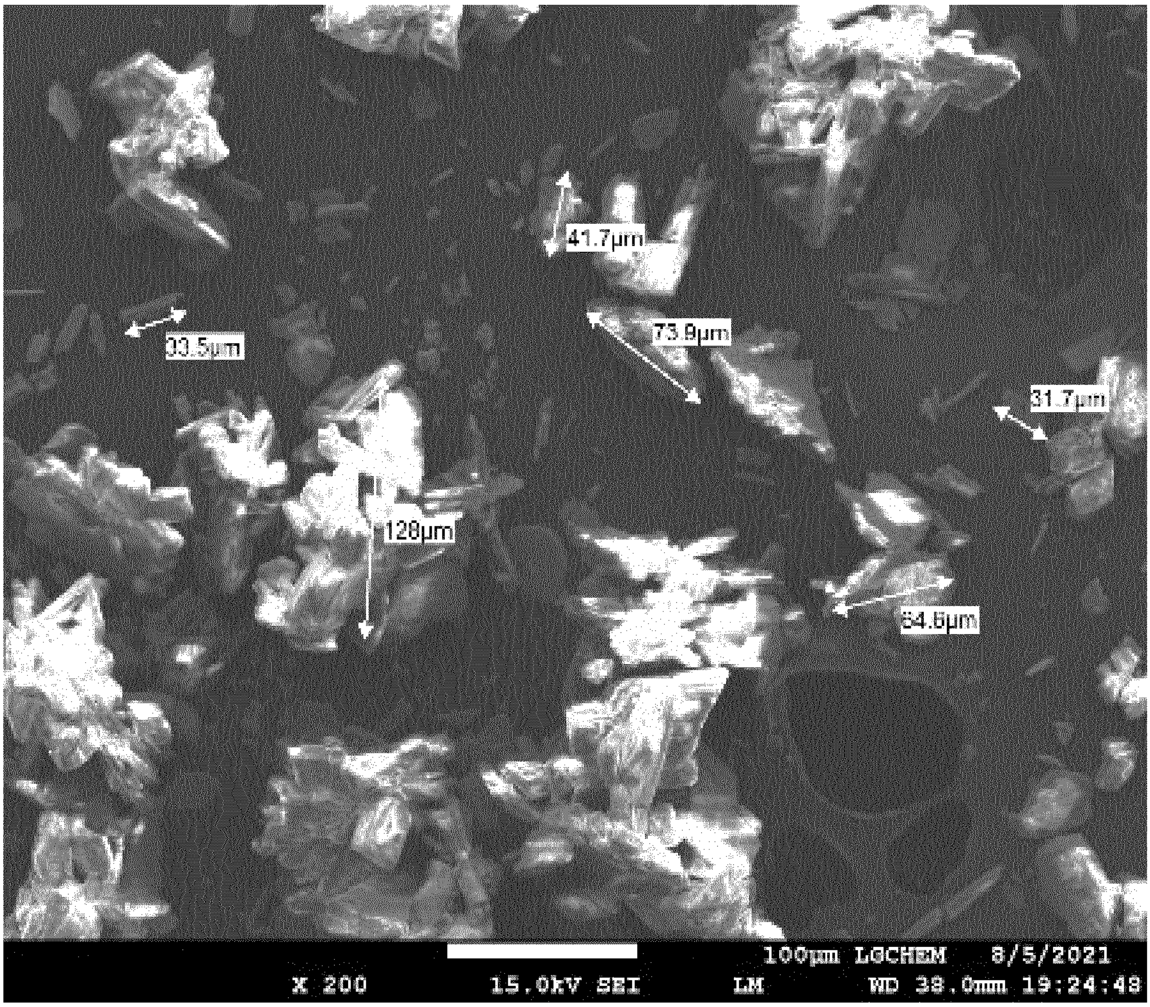
41.7μm
73.9μm
31.7μm
128μm
64.6μm
100μm LGCHEM 8/5/2021
X 200 15.0kV SEI LM WD 38.0mm 19:24:48

## INTERNATIONAL SEARCH REPORT

International application No.
**PCT/KR2022/019074**

**A. CLASSIFICATION OF SUBJECT MATTER**

**C07C 59/01**(2006.01)i; **C12P 3/00**(2006.01)i; **C12P 7/52**(2006.01)i; **C01F 11/46**(2006.01)i

According to International Patent Classification (IPC) or to both national classification and IPC

**B. FIELDS SEARCHED**

Minimum documentation searched (classification system followed by classification symbols)

C07C 59/01(2006.01); B01D 1/26(2006.01); B01D 61/02(2006.01); B01D 69/02(2006.01); C07C 51/41(2006.01); C07C 51/44(2006.01); C07C 51/48(2006.01); C07C 53/122(2006.01); C12P 7/52(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 알카리 금속염(alkali metal salt), 유기산 발효액(organic acid fermented solution), 황산염(sulfate), 황산칼슘(calcium sulfate), 결정(crystal), x-선 회절 스펙트럼(X-ray diffraction spectrum), 회수(collection)

**C. DOCUMENTS CONSIDERED TO BE RELEVANT**

| Category* | Citation of document, with indication, where appropriate, of the relevant passages | Relevant to claim No. |
|---|---|---|
| X | KR 10-2021-0012270 A (LG CHEM, LTD.) 03 February 2021 (2021-02-03)<br>See claims 11 and 12; paragraphs [0003], [0008], [0049], [0050], [0054] and [0069]; and example 1. | 1-4,8-11 |
| Y | | 5-7,12-16 |
| Y | KR 10-2008-0081347 A (BASF SE) 09 September 2008 (2008-09-09)<br>See paragraphs [0013], [0021] and [0031]-[0034]. | 5-7,12-16 |
| A | WO 2016-146721 A1 (PURAC BIOCHEM BV) 22 September 2016 (2016-09-22)<br>See entire document. | 1-16 |
| A | KR 10-2018-0036635 A (KOREA RESEARCH INSTITUTE OF CHEMICAL TECHNOLOGY) 09 April 2018 (2018-04-09)<br>See entire document. | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 March 2023** | **08 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2022/019074**

**C. DOCUMENTS CONSIDERED TO BE RELEVANT**

| Category* | Citation of document, with indication, where appropriate, of the relevant passages | Relevant to claim No. |
|---|---|---|
| A | WO 2020-137974 A1 (TORAY INDUSTRIES, INC.) 02 July 2020 (2020-07-02)<br>See entire document. | 1-16 |

Form PCT/ISA/210 (second sheet) (July 2022)

**INTERNATIONAL SEARCH REPORT**
**Information on patent family members**

International application No.
**PCT/KR2022/019074**

| Patent document cited in search report | Publication date (day/month/year) | Patent family member(s) | Publication date (day/month/year) |
|---|---|---|---|
| KR 10-2021-0012270 A | 03 February 2021 | None | |
| KR 10-2008-0081347 A | 09 September 2008 | BR PI0620816 A2 | 05 June 2012 |
| | | BR PI0620816 B1 | 08 March 2016 |
| | | CA 2632974 A1 | 12 July 2007 |
| | | CN 101351437 A | 21 January 2009 |
| | | CN 101351437 B | 10 July 2013 |
| | | DE 102005063109 A1 | 05 July 2007 |
| | | EP 1968929 A2 | 17 September 2008 |
| | | JP 2009-522237 A | 11 June 2009 |
| | | JP 5080496 B2 | 21 November 2012 |
| | | KR 10-1483798 B1 | 16 January 2015 |
| | | MY 154911 A | 28 August 2015 |
| | | NO 20082710 L | 22 August 2008 |
| | | RU 2008130981 A | 10 February 2010 |
| | | RU 2454396 C2 | 27 June 2012 |
| | | US 2008-0317934 A1 | 25 December 2008 |
| | | US 8293793 B2 | 23 October 2012 |
| | | WO 2007-077200 A2 | 12 July 2007 |
| | | WO 2007-077200 A3 | 21 December 2007 |
| WO 2016-146721 A1 | 22 September 2016 | BR 112017019487 A2 | 15 May 2018 |
| | | CA 2979586 A1 | 22 September 2016 |
| | | CL 2017002290 A1 | 16 March 2018 |
| | | CN 107438673 A | 05 December 2017 |
| | | CN 107438673 B | 24 June 2022 |
| | | CN 115181762 A | 14 October 2022 |
| | | CO 2017009093 A2 | 30 November 2017 |
| | | EP 3070173 A1 | 21 September 2016 |
| | | EP 3271466 A1 | 24 January 2018 |
| | | EP 3271466 B1 | 19 December 2018 |
| | | EP 3492598 A1 | 05 June 2019 |
| | | ES 2714848 T3 | 30 May 2019 |
| | | KR 10-2006614 B1 | 02 August 2019 |
| | | KR 10-2017-0131498 A | 29 November 2017 |
| | | KR 10-2019-0091384 A | 05 August 2019 |
| | | KR 10-2171892 B1 | 02 November 2020 |
| | | MX 2017011424 A | 17 May 2018 |
| | | MX 364049 B | 09 April 2019 |
| | | US 10815503 B2 | 27 October 2020 |
| | | US 2018-0073044 A1 | 15 March 2018 |
| | | US 2021-0002679 A1 | 07 January 2021 |
| KR 10-2018-0036635 A | 09 April 2018 | KR 10-1985444 B1 | 03 June 2019 |
| WO 2020-137974 A1 | 02 July 2020 | AR 117505 A1 | 11 August 2021 |
| | | CL 2021001577 A1 | 19 November 2021 |
| | | CN 113226520 A | 06 August 2021 |
| | | CN 113226520 B | 03 May 2022 |
| | | EP 3885031 A1 | 29 September 2021 |
| | | KR 10-2021-0107676 A | 01 September 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210167307
- KR 1020210167409
- KR 1020220161896


### Non-patent literature cited in the description


- *Econ, Environ, Geol.,* 2011, vol. 44 (1), 37-48